(19) Europäisches Patentamt / European Patent Office / Office européen des brevets

(11) **EP 2 147 687 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
*A61L 27/24* *(2006.01)*    *A61L 27/36* *(2006.01)*

(21) Anmeldenummer: **09014319.9**

(22) Anmeldetag: **21.07.2008**

(54) **Flächiges Implantat**

Laminar implant

Implant plat

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **24.07.2007 DE 102007037051**

(43) Veröffentlichungstag der Anmeldung:
**27.01.2010 Patentblatt 2010/04**

(62) Dokumentnummer(n) der früheren Anmeldung(en) nach Art. 76 EPÜ:
**08013085.9 / 2 018 880**

(73) Patentinhaber: **Aesculap AG**
**78532 Tuttlingen/Donau (DE)**

(72) Erfinder:
• **Odermatt, Erich**
**CH-8200 Schaffhausen (DE)**
• **Wegmann, Jürgen**
**78333 Stockach (DE)**
• **Blender, Bernd**
**88367 Hohentengen (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
**EP-A- 1 738 780        WO-A-99/13902**
**DE-A1-102005 054 940    US-A1- 2002 013 627**
**US-A1- 2007 073 415**

• **DATABASE MEDLINE [Online] US NATIONAL LIBRARY OF MEDICINE (NLM), BETHESDA, MD, US; September 2002 (2002-09), BAHARUDDIN A ET AL: "Bovine pericardium for dural graft: clinical results in 22 patients." XP002518556 Database accession no. NLM12140102 & CLINICAL NEUROLOGY AND NEUROSURGERY SEP 2002, Bd. 104, Nr. 4, September 2002 (2002-09), Seiten 342-344, ISSN: 0303-8467**

**Beschreibung**

Die Erfindung betrifft ein flächiges Implantat mit einem schichtförmigen Aufbau, welches sich insbesondere zum Ersatz von biologischen Geweben eignet.

Unter der Hirnhaut werden die Bindegewebsschichten verstanden, die das Gehirn umgeben und in die Rükkenmarkshaut übergehen, welche das übrige zentrale Nervensystem (ZNS) umgibt. Die sogenannte Dura Mater stellt dabei die äußerste Hirnhaut dar. Sie besteht aus einem überwiegend straffen und collagenfaserigen Bindegewebe und hat vor allem die Funktion einer Organkapsel, wobei sie insbesondere einen Austritt der cerebrospinalen Flüssigkeit verhindert.

Die Dura Mater ist in zwei Blätter unterteilt, wobei im Bereich des Schädels das äußere Blatt identisch mit der Knochenhaut ist. Dadurch sind beispielsweise bei neurochirurgischen Eingriffen Verletzungen der Dura Mater und insbesondere des darunter liegenden Weichgewebes möglich. Die Verletzungen der Dura Mater (äußerste Hirnhaut) können in diesen Fällen so schwerwiegend sein, dass ein Ersatz zumindest von Teilen der Dura Mater erforderlich ist. Da die Dura Mater zudem sehr stark von Blutgefäßen durchzogen ist, sind derartige Verletzungen häufig von starken Blutungen begleitet.

Aufgabe der vorliegenden Erfindung ist es somit, ein Implantat bereitzustellen, welches insbesondere für den Ersatz der Dura Mater verwendet werden kann und insbesondere gute hämostatische sowie gewebehaftende Eigenschaften aufweist.

Diese Aufgabe wird gelöst durch ein flächiges Implantat, insbesondere zum Ersatz von biologischen Geweben, mit einem schichtförmigen Aufbau mit, vorzugsweise aus,

a) einer lyophilisierten Perikardschicht biologischen Ursprungs und
b) mindestens einer schwammartigen Schicht aus lyophilisiertem extrazellulären Protein.

Durch die Erfindung wird ein Implantat bereitgestellt, welches infolge seines kompositären Aufbaus sowohl die vorteilhaften Eigenschaften eines Perikards als auch eines Proteinschwamms aufweist. Entsprechend der natürlichen Beschaffenheit eines Perikards weist die Perikardschicht des Implantats in der Regel eine lederartige und somit mechanisch stabile Beschaffenheit auf. Die Perikardschicht dient vorzugsweise als eine Art stabilisierende Unterlage bzw. Trägermaterial für die schwammartige Schicht. Darüber hinaus hat die Pericardschicht den Vorteil, dass sie im Wesentlichen keine adhäsiven Eigenschaften besitzt und somit auch keine Gewebeverwachsungen, beispielsweise mit dem Schädelknochen, verursacht. Die schwammartige Proteinschicht besitzt bevorzugterweise gewebeverklebende Eigenschaften. Dadurch kann das Implantat direkt auf das zu versorgende Gewebe aufgelegt werden, ohne dass zusätzliche Fixierungsschritte, wie beispielsweise ein Annähen, erforderlich sind. Mit anderen Worten besitzt das Implantat in vorteilhafter Weise selbstklebende Eigenschaften. Die Haftung des Implantats auf Gewebeoberflächen ist dabei vor allem auf die Kapillarkräfte der schwammartigen Proteinschicht zurückzuführen. Die Feuchtigkeit des Gewebes dient dabei als Haftermittler. Die Verwendung des erfindungsgemäßen Implantats kann daher auch einen Zeitgewinn für den Chirurgen bewirken. Darüber hinaus erlaubt die robuste und mechanisch stabile Perikardschicht im Bedarfsfall auch ein Annähen des Implantats an der Versorgungs- bzw. Applikationsstelle im menschlichen oder tierischen Körper. So läßt sich das Implantat beispielsweise an Hartgewebe, insbesondere Knochengewebe, annähen. Das Implantat kann weiterhin in vorteilhafter Weise ein Mehrfaches seines Eigengewichtes an Flüssigkeiten aufnehmen. Dadurch eignet sich das erfindungsgemäße Implantat auch zur blutstillenden Versorgung von Wunden, indem Blut und gegebenenfalls auch Exsudat aus dem Wundbereich durch die schwammartige Proteinschicht schnell und nachhaltig aufgesaugt werden können. Gleichzeitig stellt die Perikardschicht wegen ihrer im Normalfall lederartigen Beschaffenheit eine unüberwindbare Flüssigkeitsbarriere dar, so dass mit Hilfe des erfindungsgemäßen Implantats auch ein dichter Verschluss der Applikationsstelle im menschlichen und/oder tierischen Körper möglich ist. Dies ist insbesondere bei der Abdichtung der Dura Mater von Vorteil, um das Auftreten von cerebrospinalen Flüssigkeits-Leckagen (CSF-Leckagen) zu vermeiden.

Die Formulierung "schwammartige Schicht aus lyophilisiertem extrazellulären Protein" bedeutet im Sinne der vorliegenden Erfindung, dass die schwammartige Schicht aus einem lyophilisierten extrazellulären Protein oder mehreren lyophilisierten extrazellulären Proteinen gebildet ist und neben dem extrazellulären Protein bzw. extrazellulären Proteinen weitere Bestandteile, wie sie beispielsweise im Folgenden noch beschrieben werden, aufweisen kann.

Das Implantat besitzt vorzugsweise einen mindestens zweischichtigen Aufbau. Das Implantat kann insbesondere in Form einer Folie vorliegen.

In einer bevorzugten Ausführungsform ist das Perikard xenogenen, insbesondere bovinen, porcinen oder equinen Ursprungs. Gewöhnlich handelt es sich bei dem Perikard um ein Rinderperikard.

Weiterhin kann auch das extrazelluläre Protein xenogenen, insbesondere bovinen, porcinen oder equinen, Ursprungs sein. Proteine equinen Ursprungs sind wegen der in diesem Fall besonders geringen Infektionsrisiken besonders bevorzugt. Weiterhin kann es sich bei dem extrazellulären Protein um ein faserförmiges Stützprotein handeln. Beispielsweise kann es sich bei dem extrazellulären Protein um Collagen und/oder Elastin handeln. Vorzugsweise ist das extrazelluläre Protein Collagen. Das Collagen kann insbesondere vom Typ I, II, III und/oder IV sein. Bevorzugt ist Collagen vom Typ I. Weiterhin kann es sich bei dem Collagen um Sehnencollagen handeln.

In einer weiteren Ausführungsform handelt es sich bei dem extrazellulären Protein um ein Plasmaprotein. Als Plasmaproteine kommen grundsätzlich Albumine und/oder Globuline in Frage, wobei es sich im Falle der Globuline vor allem um $\alpha$1-Globuline, $\alpha$2-Globuline und/oder $\beta$-Globuline handeln kann. Bei dem extrazellulären Protein kann es sich weiterhin auch um ein blutgerinnungshemmendes Protein, insbesondere Makroglobulin und/oder Antithrombin, handeln. Des Weiteren kommt Fibrinogen, welches die Vorstufe von monomerem Fibrin darstellt, in Betracht.

In einer weitergehenden Ausführungsform liegt das extrazelluläre Protein in denaturierter Form vor. Beispielsweise kann das extrazelluläre Protein in Form eines Teilhydrolysats vorliegen. Bevorzugt handelt es sich bei dem extrazellulären Protein um Gelatine. Die Gelatine besitzt vorzugsweise ein Molekulargewicht (MG) von 100 bis 500 kDa (Kilo Dalton), insbesondere von 150 bis 250 kDa. Beispielsweise kann es sich bei dem extrazellulären Protein um teilhydrolysierte Gelatine mit einem Molekulargewicht von bis zu 100 kDa handeln. Die Gelatine kann beispielsweise durch eine Bloomzahl von ca. 240 charakterisiert sein. Gelatine besitzt außerdem den Vorteil, dass sie besonders gut auf der Gewebeoberfläche haftet.

Erfindungsgemäß ist es weiterhin möglich, dass das extrazelluläre Protein rekombinanten Ursprungs ist. Beispielsweise kann das extrazelluläre Protein durch Mikroorganismen, insbesondere Hefezellen, hergestellt sein.

In einer bevorzugten Ausführungsform stellt das extrazelluläre Protein eine Mischung von verschiedenen Proteinen dar, d.h. es handelt sich bei der schwammartigen Proteinschicht um eine Schicht aus mehreren lyophilisierten extrazellulären Proteinen. Beispielsweise kann es sich bei der Proteinmischung um eine Mischung aus Gelatine und Collagen handeln. Dadurch können die guten Hafteigenschaften von Gelatine sowie die stabilen Fasereigenschaften von Collagen in besonders vorteilhafterweise in dem erfindungsgemäßen Implantat verwirklicht werden.

Das extrazelluläre Protein ist unvernetzt. Vorzugsweise ist die schwammartige Schicht frei von Vernetzungsmitteln. Dies hat den Vorteil, daß die schwammartige Schicht und damit das Implantat insgesamt schneller abbaubar bzw. resorbierbar ist.

Grundsätzlich können beide Seiten der Perikardschicht mit der schwammartigen Schicht belegt sein. Bevorzugt ist die Perikardschicht einseitig mit der schwammartigen Schicht belegt. In dieser Ausführungsform kann mit besonderem Vorteil insbesondere die mechanische Stabilität der Perikardschicht mit den vor allem gewebeadhäsiven Eigenschaften der schwammartigen Proteinschicht kombiniert werden.

Um eine möglichst feste Verbindung zwischen der Perikard- und Proteinschicht zu erhalten, ist in einer weiteren Ausführungsform die der schwammartigen Schicht zugewandte Oberfläche der Perikardschicht aufgerauht. Vorzugsweise wird das Perikard nach dessen Lyophilisation aufgerauht. Die Aufrauhung der Perikardoberfläche erfolgt zweckmäßigerweise vor der Auflyophilisation des Proteinschwammes auf das Perikard. Die Aufrauhung kann mechanisch, insbesondere mit Hilfe einer Bürste oder eines Nadelbrettes, vorgenommen werden. Daneben können auch andere Verfahren zur Behandlung, insbesondere Verfahren zur Aktivierung, der Perikardoberfläche eingesetzt werden. Beispielsweise kann die Oberfläche der Perikardschicht durch Plasmabehandlung aktiviert sein.

Gemäß einer weiteren Ausführungsform ist die schwammartige Schicht auf die Perikardschicht als Unterlage auflyophilisiert. Zu diesem Zweck wird das vorzugsweise zuvor separat lyophilisierte Perikard gewöhnlich in eine formgebende Umgebung, beispielsweise in eine Lyophilisationsschale, vorgelegt, mit einer Dispersion des extrazellulären Proteins übergossen und anschließend lyophilisiert. Die Proteindispersion liegt bevorzugt als wässrige Suspension vor, welche gegebenenfalls mit einem Alkohol, beispielsweise Isopropanol, zur Erhöhung der Proteinlöslichkeit versetzt sein kann. Alternativ kann eine feuchte oder nicht vollständig getrocknete Pericardschicht in eine formgebende Umgebung, beispielsweise Lyophilisationsschale, vorgelegt werden und mit einer schwammartigen Schicht eines zuvor lyophilisierten extrazellularen Proteins bedeckt bzw. belegt werden. Eine anschließende Lyophilisation führt zu einem erfindungsgemäßen Implantat.

In einer alternativen Ausführungsform ist die schwammartige Schicht mit der Perikardschicht als Unterlage chemisch fixiert. Die chemische Fixierung kann beispielsweise durch geeignete Vernetzungsmittel erreicht werden. Bezüglich der Eigenschaften und Merkmale der in Frage kommenden Vernetzungsmittel wird auf die bisherige Beschreibung Bezug genommen.

Das erfindungsgemäße Implantat besitzt vorzugsweise eine Dicke von 1 bis 10 mm, insbesondere von 2 bis 5 mm. Die Perikardschicht besitzt insbesondere eine Dicke von 0,1 bis 4 mm, vorzugsweise 0,5 bis 2 mm. Die schwammartige Schicht aus dem extrazellulären Protein besitzt bevorzugt eine Schichtdicke von 1 bis 10 mm, insbesondere 2 bis 5 mm, vorzugsweise 3 bis 4 mm.

In einer weitergehenden Ausführungsform besitzt das erfindungsgemäße Implantat ein Flächengewicht von 80 bis 480 g/m$^2$, insbesondere 160 bis 330 g/m$^2$. Das Flächengewicht der Perikardschicht liegt bevorzugterweise zwischen 50 und 180 g/m$^2$, insbesondere 80 und 110 g/m$^2$. Die schwammartige Schicht besitzt vorzugsweise ein Flächengewicht von 30 bis 300 g/m$^2$, insbesondere 80 bis 120 g/m$^2$. Die Dichte des Implantats liegt bevorzugt zwischen 30 und 160 g/dm$^3$, insbesondere zwischen 50 und 110 g/dm$^3$.

In einer weiteren Ausführungsform weist das Implantat überstehende Ränder auf. Erfindungsgemäß kann die Perikard- oder die schwammartige Proteinschicht im Implantat überstehende Ränder aufweisen. Auf diese Weise können abhängig vom Einsatzzweck des Implantats entweder mehr die Eigenschaften der Perikardschicht, insbesondere deren mechanische Stabilität, oder die Eigenschaften der schwammartigen Proteinschicht, vorzugsweise deren gewebeverklebenden Eigenschaften, im Implantat hervorgehoben werden.

Die schwammartige Proteinschicht ist bevorzugt vollflächig auf der Perikardoberfläche ausgebildet.

In einer möglichen Ausführungsform ist die schwammartige Schicht nur teilweise auf der Oberfläche der Perikardschicht ausgebildet. Erfindungsgemäß kann es zum Beispiel vorgesehen sein, dass sich die Proteinschicht nur punktweise oder am Außenrand auf der Perikardschicht befindet.

Weiterhin ist es bevorzugt, dass die schwammartige Schicht mindestens eine wasserlösliche bioverträgliche organische Säure enthält. Der Säuregehalt in der schwammartigen Proteinschicht liegt bevorzugterweise zwischen 1 und 10 Gew.-%, insbesondere zwischen 2 und 8 Gew.-%. Bei der organischen Säure handelt es sich zweckmäßigerweise um eine nicht flüchtige Säure. Die organische Säure kann insbesondere eine niedermolekulare Säure sein: Bevorzugt ist die Säure eine aliphatische Säure. Beispielsweise kann die Säure eine Kohlenstoffkette mit 1 bis 6 Kohlenstoffatomen, vorzugsweise 3 bis 6 Kohlenstoffatomen, aufweisen. Die Säure kann weiterhin eine mehrwertige, insbesondere zweiwertige, Säure sein. Weiterhin kann es sich bei der Säure um eine Hydroxycarbonsäure, insbesondere mehrwertige Hydroxycarbonsäure, handeln. Beispielsweise kann es sich bei der Säure um eine Zuckersäure handeln.

Vorzugsweise handelt es sich bei der wasserlöslichen bioverträglichen organischen Säure der schwammartigen Proteinschicht um eine Säure aus der Gruppe, umfassend Zitronensäure, Weinsäure, Ascorbinsäure, Apfelsäure, Gluconsäure, Schleimsäure, Glutarsäure und Adipinsäure. Zitronensäure ist besonders bevorzugt.

Der Säurezusatz in der schwammartigen Proteinschicht verbessert mit besonderem Vorteil deren Aufnahmevermögen für Flüssigkeiten, insbesondere für Körperflüssigkeiten, vorzugsweise für Blut. Dadurch kann insgesamt das Flüssigkeitsaufnahmevermögen des Implantats erhöht werden. Bevorzugt weist die schwammartige Schicht ein Flüssigkeitsaufnahmevermögen auf, das dem 20- bis 60-fachen, insbesondere 30- bis 60-fachen, seines Eigengewichtes entspricht. In dieser Ausführungsform eignet sich das erfindungsgemäße Implantat in besonderer Weise für die Blutstillung (Hämostase).

In einer weiteren Ausführungsform ist die schwammartige Schicht innerhalb eines Zeitraums < 100 Sekunden, insbesondere < 60 Sekunden, vollständig, d. h. an seiner äußeren und inneren Oberfläche, mit Wasser benetzbar. In Bezug auf Blut ist das Implantat vorzugsweise innerhalb eines Zeitraums < 180 Sekunden vollständig benetzbar.

Die schwammartige Schicht besitzt gemäß einer weiteren Ausführungsform bei Zusatz von Wasser einen pH-Wert < 4. Vorzugsweise besitzt die schwammartige Proteinschicht in Wasser einen pH-Wert zwischen 3,0 und 3,5.

Erfindungsgemäß ist es weiterhin vorgesehen, dass das Implantat, insbesondere die schwammartige Proteinschicht, Wirkstoffe enthält. Die Wirkstoffe können insbesondere antimikrobielle Eigenschaften aufweisen. Beispielsweise kann es sich bei den Wirkstoffen um Antibiotika handeln. Bei den Antibiotika kann es sich um Gentamycin und/oder Rifampicin handeln. Weiterhin können als Wirkstoffe bioverträgliche Metallverbindungen, insbesondere Metallsalze, vorzugsweise Silbersalze, in Betracht kommen. Beispielsweise kann das Implantat Silberacetat enthalten. Weiterhin kann es sich bei den Wirkstoffen auch um Metalle in elementarem Zustand handeln, welche vorzugsweise in Form von Nanopartikeln vorliegen. So kann das erfindungsgemäße Implantat zum Beispiel Silbernanopartikel enthalten.

Bei den Wirkstoffen kann es sich weiterhin um entzündungshemmende Verbindungen, insbesondere um Allatoin, Saponin, Riboflavin, Flavonoide, Tocopherol, Beta-Sitosterol, Soledum-Cineol, Dexpanthenol und/oder Bromalain, handeln. Als Flavonoide kommen bevorzugt Nobiletin, Rutin und/oder Hesperidin in Betracht.

In einer weiteren bevorzugten Ausführungsform weist das Implantat mindestens eine gefärbte Schicht auf. Vorzugsweise ist die schwammartige Schicht aus dem extrazellulären Protein mit mindestens einem Farbstoff gefärbt. Bei dem Farbstoff kann es sich beispielsweise um D- & C-Farbstoffe (Drug- und Cosmetic-Farbstoffe), Riboflavin, Retinol und/oder Methylenblau handeln. Die Schichtfärbung des Implantats kann vor allem für eine verbesserte Erkennbarkeit seiner Schichten, insbesondere für eine erleichterte Unterscheidbarkeit der schwammartigen Proteinschicht von der Perikardschicht, nützlich sein.

Das erfindungsgemäße Implantat liegt zweckmäßigerweise in sterilisierter Form vor. Als Sterilisationsmethoden kommen grundsätzlich alle fachmännischen Verfahren, beispielsweise γ-Sterilisierung, ElektronenBestrahlung, Ethylenoxidbegasung oder Plasmasterilisation in Frage. Das Implantat kann zudem in konfektionierter Form vorliegen.

Die vorliegende Erfindung betrifft auch ein Verfahren zur Herstellung des Implantats, wobei eine Perikardschicht und zumindest eine Schicht aus extrazellulärem Protein unter Verbindung miteinander lyophilisiert werden.

In einer bevorzugten Ausführungsform umfaßt das Verfahren die folgenden Schritte:

a) Aufbringen einer flüssigen Dispersion, umfassend ein extrazelluläres Protein und zumindest ein flüssiges Dispersionsmittel, auf eine Perikardschicht,

b) Abkühlen und Verfestigen der aufgebrachten Dispersion unter Ausbildung mindestens einer festen Schicht, umfassend das extrazelluläre Protein und das zumindest eine Dispersionsmittel,

c) Entfernen des Dispersionsmittels durch Lyophilisation unter Verbindung der Schichten miteinander.

Bevorzugt wird die Perikardschicht vor dem Aufbringen der Dispersion lyophilisiert. Dabei kann die Perikardschicht nach ihrer Lyophilisation aufgerauht werden. Diesbezüglich wird auf die bisherige Beschreibung Bezug genommen.

Die flüssige Dispersion kann in Form einer Lösung oder Suspension bereitgestellt werden. Als Dispersionsmittel wird bevorzugt Wasser verwendet, welches gegebenenfalls mit einem Alkohol, insbesondere Isopropanol, versetzt werden kann, um die Löslichkeit des extrazellulären Proteins zu erhöhen.

Für das Aufbringen der flüssigen Dispersion auf die Perikardschicht kann letztere in eine formgebende Umgebung, beispielsweise in eine Lyophilisationsschale, überführt werden und mit der flüssigen Dispersion übergossen werden. Alternativ dazu kann zuerst die flüssige Dispersion in eine formgebende Umgebung überführt werden. In diesem Fall wird die Perikardschicht dann auf die Dispersion aufgelegt. In beiden Fällen schließt sich danach die Abkühlung und Verfestigung der flüssigen Dispersion sowie die Entfernung des flüssigen Dispersionsmittels durch Lyophilisation an.

Bevorzugt wird zur Herstellung des Implantats eine flüssige Dispersion mit einem Anteil an dem extrazellulären Protein zwischen 0,1 und 5 Gew.-%, insbesondere 0,2 und 2,5 Gew.-%, bezogen auf das Gesamtgewicht der flüssigen Dispersion, verwendet. Handelt es sich bei dem extrazellulären Protein um Gelatine, so wird bevorzugt eine Dispersion mit einem Gelatineanteil zwischen 0,2 und 0,5 Gew.-%, bezogen auf das Gesamtgewicht der Dispersion, verwendet. Im Falle von Kollagen als extrazellulärem Protein wird bevorzugt mit einer Kollagendispersion gearbeitet, deren Anteil an Kollagen zwischen 2 und 2,5 Gew.-%, bezogen auf das Gesamtgewicht der Dispersion, liegt. Die Lyophilisation wird vorzugsweise in einem Temperaturbereich zwischen -10 und -50 °C, insbesondere -20 und -40 °C, vorgenommen.

In einer alternativen Ausführungsform wird eine feuchtete oder noch nicht vollständig getrocknete Perikardschicht mit einer bereits lyophilisierten Schicht des extrazellulären Proteins belegt bzw. beschichtet und anschließend einer Lyophilisation unter Verbindung der beiden Schichten unterworfen. Zu diesem Zweck kann die Pericardschicht vor der Belegung bzw. Beschichtung mit dem extrazellulären Protein mit Wasser angefeuchtet werden. Bezüglich weiterer Merkmale und Einzelheiten wird auf die bisherige Beschreibung verwiesen.

Das Implantat eignet sich allgemein mit besonderem Vorteil zur Versorgung, insbesondere zum Ersatz und/oder Verschluss, von Gewebedefekten, vorzugsweise Weichgewebedefekten. Bevorzugt wird das Implantat zur Versiegelung bzw. Abdichtung von Flüssigkeits- und/oder Luftleckagen im menschlichen und/oder tierischen Körper verwendet.

Bevorzugt wird das Implantat zum Ersatz und/oder Verschluss der Dura Mater verwendet, wobei das Implantat vorzugsweise als Dura Mater-Onlay ausgebildet ist. Das Implantat kann insbesondere zur Abdichtung einer verletzten Dura Mater verwendet werden, ohne dass hierzu vorher Dura Mater-Gewebe des Patienten entfernt wird. Das Implantat eignet sich insbesondere zur Abdichtung der cerebrospinalen Flüssigkeit des Gehirns. Diese abdichtenden Eigenschaften des Implantats können vor allem durch seine Perikardschicht erreicht werden, welche allgemein gegenüber Flüssigkeiten, insbesondere Körperflüssigkeiten, eine Art absolute physikalische Flüssigkeitsbarriere darstellt. Im Gegensatz dazu besteht bei herkömmlichen reinen Proteinschwämmen die Gefahr, dass bei Überschreitung von deren maximaler Aufnahmekapazität für Flüssigkeiten weitere Flüssigkeitsmengen nicht mehr zurückgehalten werden können. Außerdem kann das Implantat, sofern erwünscht bzw. erforderlich, über die Perikardschicht mit der Dura Mater vernäht werden.

Weiterhin kann das erfindungsgemäße Implantat, insbesondere wegen der gewebeverklebenden Eigenschaften seiner schwammartigen Proteinschicht, auch als blutstillendes Mittel oder Hämostyptikum bzw. Hämostatikum verwendet werden. In dieser Form kann das Implantat zur blutstillenden Versorgung von inneren Wunden, insbesondere Parenchymorganen, wie zum Beispiel der Leber, der Milz, des Pankreas, der Nieren, der Lunge, der Nebennieren, aber auch zur Versorgung der Schilddrüse und/oder Lymphknoten verwendet werden. Das Implantat eignet sich weiterhin auch für thorakale Eingriffe, beispielsweise zum Verschluss oder zur Abdichtung von Luftleckagen in der Lunge. Ebenso ist ein Einsatz des Implantats bei gastrointestinalen Operationen, insbesondere zur Abdichtung von Darmanastomosen, möglich. Eine weitere Verwendungsmöglichkeit des Implantats besteht in der Abdeckung von Urethra-Erosionen. Bei den vorstehend genannten Anwendungen kann das Implantat aufgrund der mechanisch stabilen, insbesondere reißfesten, Beschaffenheit der Perikardschicht mit besonderem Vorteil auf die jeweilige Wunde aufgenäht werden. Dies ist bei einem Implantat, welches ausschließlich aus Collagen besteht, nicht möglich.

Die vorliegende Erfindung umfaßt auch die Verwendung von a) einer lyophilisierten Perikardschicht biologischen Ursprungs und b) mindestens einer schwammartigen Schicht mit, vorzugsweise aus, lyophilisiertem extrazellulären Protein zur Herstellung eines flächigen Implantats. Bezüglich weiterer Einzelheiten und Merkmale zu dem Implantat, der Perikardschicht, der schwammartigen Proteinschicht sowie den medizinischen Verwendungsmöglichkeiten des Implantats wird auf die bisherige Beschreibung Bezug genommen.

Weitere Einzelheiten und Merkmale der Erfindung ergeben sich aus der nachfolgenden Beschreibung bevorzugter Ausführungsformen in Form von Figuren, Beispielen und Unteransprüchen. In diesen Ausführungsformen können einzelne Merkmale der Erfindung allein oder in Kombination mit anderen Merkmalen verwirklicht sein. Sämtliche Figuren werden hiermit durch ausdrückliche Bezugnahme zum Inhalt dieser Beschreibung gemacht.

In den Figuren ist Folgendes gezeigt:

Figur 1: schematische Draufsicht auf ein erfindungsgemäßes Im- plantat mit überstehenden Rändern der Perikardschicht,

Figur 2: schematische Draufsicht auf ein erfindungsgemäßes Implantat mit überstehenden Rändern der schwammartigen Proteinschicht,

Figur 3: schematische Ansicht eines erfindungsgemäßen Implantats.

Figurenbeschreibung

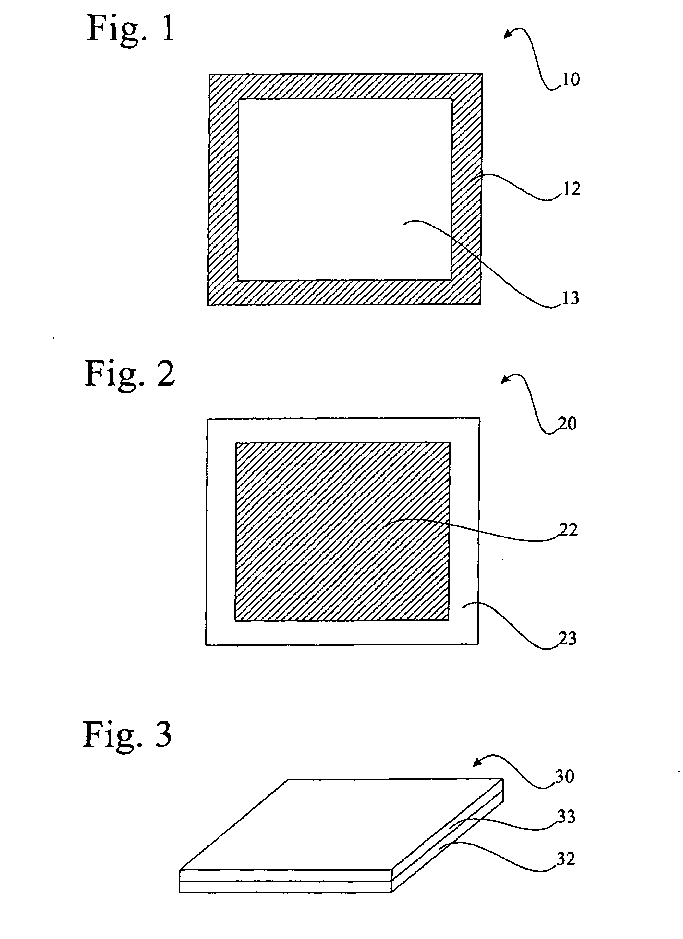

Figur 1 zeigt schematisch die Draufsicht auf ein erfindungsgemäßes Implantat 10 mit einem zweischichtigen Aufbau aus einer lyophilisierten Perikardschicht 12 und einer schwammartigen Schicht 13 aus lyophilisiertem extrazellulären Protein 13. Die Perikardschicht 12 weist im Implantat 10 überstehende Ränder auf. Die flächenmäßig größere Perikardschicht 12 verleiht dem Implantat 10 besonders stabile Eigenschaften. Beispielsweise können die überstehenden Ränder der Perikardschicht 12 zum Aufnähen des Implantats 10 auf eine Wunde dienen.

Figur 2 zeigt schematisch die Draufsicht auf ein erfindungsgemäßes Implantat 20 mit einem zweischichtigen Aufbau aus einer lyophilisierten Perikardschicht 22 und einer schwammartigen Schicht 23 aus lyophilisiertemj extrazellulären Protein. Die schwammartige Proteinschicht 23 weist im Implantat 20 überstehende Ränder auf. Dadurch können insbesondere die gewebeverklebenden Eigenschaften des Implantats 20 verbessert werden.

Figur 3 zeigt schematisch ein erfindungsgemäßes Implantat 30 mit einem zweischichtigen Aufbau aus einer lyophilisierten Pericardschicht 32 und einer schwammartig ausgebildeten Schicht 33 aus lyophilisiertem extrazellulären Protein.

Beispiel 1:

Herstellung von erfindungsgemäßen Implantaten

a) Gefriergetrocknetes Rinderperikard wurde auf eine Größe von 10 x 14 cm zugeschnitten und mit einer Bürste aufgerauht. Danach wurden 6,6 g Collagen in 132 ml Reinstwasser (milliQ-Wasser der Firma Millipor, Deutschland), welches Zusätze an Essigsäure enthielt, gequollen. Das gequollene Collagen wurde während 20 Minuten in einem Lösungsmittelgemisch aus 231 ml Reinstwasser und 33 ml Isopropanol suspendiert. Anschließend wurden 264 mg Äpfelsäure (0,1 Gew.-%, bezogen auf das Gesamtgewicht der Collagensuspension) in 264 ml der Suspension gelöst. Anschließend wurden jeweils 65 g der Suspension auf Lyophilisationsschalen mit einer Grundfläche von 165 cm$^2$ gegossen. Die zuvor zugeschnittenen Rinderperikardstücke wurden mit der aufgerauhten Seite auf die Suspension aufgelegt. Danach wurde die Zusammensetzung bei -40 °C tiefgefroren und lyophilisiert. Als Produkte wurden rechteckige Platten aus mit einem Collagenschwamm beschichteten Rinderperikard erhalten, wobei der Schwamm mechanisch fest auf dem Rinderperikard aufgebracht war und diese kompositäre Verbindung insbesondere beim Eintauchen in Wasser stabil blieb.

b) Es wurden 2,64 g Eisessig und 6,6 g Collagen in 132 ml Reinstwasser (MilliQ, Firma Millipore, Deutschland) eingewogen. Man ließ das Collagen insgesamt während 16 Stunden quellen. Danach wurde das gequollene Collagen mehrfach mit Reinstwasser gewaschen und während 20 Minuten in einem Gemisch aus 231 ml Reinstwasser und 33 ml Isopropanol suspendiert. Jeweils 65 g der Suspension wurden in eine Lyophilisationsschale mit einer Grund- fläche von 165 cm$^2$ gegossen. Anschließend wurde lyophilisiert. Die nach der Lyophilisation erhaltenen Collagenplatten wurden aus den Schalen entnommen und bis zur Weiterverarbeitung trocken gelagert.
Aufgearbeitete und entfettete Rinderperikardstücke wurden mit Reinstwasser angefeuchtet und in die zuvor beschriebenen Lyophilisationsschalen vorgelegt. Danach wurden die Collagenplatten auf das Perikard aufgelegt. Die Schalen wurden anschließend eingefroren und lyophilisiert. Als Produkte wurden rechteckige, mit einem Collagenschwamm beschichtete Rinderperikard-Stücke erhalten, wobei der Schwamm fest auf dem Rinderperikard aufgebracht war. Beim Eintauchen in Wasser blieb der kompositäre Aufbau des Implantats erhalten.

In einem Parallelversuch wurden reine Collagenschwämme, d.h. ohne Perikard, durch Lyophilisation hergestellt.

Beispiel 2:

Verhalten gegenüber heparinisiertem Blut

Die in Beispiel 1 hergestellten Implantate wurden in 3 x 5 cm große Stücke zugeschnitten. In einer Kristallisationsschale wurden 150 ml frisches heparinisiertes Schweineblut vorgelegt. Auf die Blutoberfläche wurden zeitgleich ein erfindungsgemäßes Implantat sowie ein reiner Collagenschwamm aufgelegt, wobei im Falle des erfindungsgemäßen Implantats die schwammartige Proteinschicht direkt auf der Blutoberfläche auflag. Anschließend wurde die Zeit bis zur vollständigen Durchdringung der beiden zu vergleichenden Implantate bestimmt. Während der reine Collagenschwamm nach 50 Sekunden vollständig mit Blut durchtränkt war, konnte im Falle des erfindungsgemäßen Implantats beobachtet werden, dass selbst nach 60 Minuten immer noch kein Blut durch das Rinderperikard durchgedrungen war. Diese Beobachtung stützt die abdichtende Wirkung der Perikardkomponente des erfindungsgemäßen Implantats gegenüber Körperflüssigkeiten, insbesondere Blut.

Das erfindungsgemäße Implantat wurde anschließend gewogen. Dabei konnte festgestellt werden, dass das Implantat die 10-fache Menge seines Eigengewichtes an Blut aufgenommen hatte. Bezogen auf den Collagenschwamm allein (abzüglich des Gewichtes des Perikards) entsprach die aufgenommene Menge an Blut dem 27-fachen des Eigengewichtes des auf das Perikard aufgebrachten Collagenschwammes. Dies verdeutlicht die hohe Saugkraft des Collagenschwammes in dem erfindungsgemäßen Implantat.

Beispiel 3:

In vitro-Untersuchungen:

3.1 Bestimmung der Stempeldurchpreßkraft

Aus Lyoplant® (gefriergetrocknetes Rinderperikard B/Braun Aesculap AG, Tuttlingen) und nach Beispiel 1) hergestellten kompositären Implantaten wurden kreisrunde Stücke mit einem Durchmesser von 45 mm ausgeschnitten. Die Stücke wurden in einer Halterung mit 25 mm Einspanndurchmesser befestigt. Zur Bestimmung der Stempeldurchpreßkraft wurde ein Stempel (Durchmesser 12,5 mm) mit einer Geschwindigkeit von 50 mm/min zentral durch die eingespannten Prüfstücke gepreßt, wobei die aufzuwendende Kraft detektiert wurde. Die in Tabelle 3.1 dargestellten Ergebnisse zeigen, dass bei dem Produkt Lyoplant® und den nach Beispiel 1 hergestellten Implantaten im wesentlichen die gleichen Durchpreßkräfte aufgewandt werden mußten. Dies verdeutlicht, daß die mechanische Stabilität bzw. Festigkeit des Perikards im erfindungsgemäßen Implantat erhalten bleibt.

Tabelle 3.1: Vergleich der mittleren Stempeldurchpreßkraft

|  | Stempeldurchpreßkraft | Standardabweichung |
|---|---|---|
| Implantat nach Bsp. 1 | 145 N | 22 N |
| Lyoplant® | 164 N | 31 N |

3.2 Fadenausreißkraft

Aus Lyoplant® (gefriergetrocknetes Rinderperikard B/Braun Aesculap AG, Tuttlingen) und den nach Beispiel 1 hergestellten kompositären Implantaten wurden rechteckige Streifen mit einer Länge von 30 mm und einer Breite von 25 mm zugeschnitten. Danach wurde ein Safil® - Faden (B/Braun, Aesculap, Tuttlingen) der Stärke USP 2,0 1 cm unterhalb der Schnittkante durch das Implantat gezogen und das Implantat in die untere Klemmbacke einer Zugprüfmaschine (Firma Zwick Roell, Ulm, Deutschland) eingespannt. Danach wurde der Faden in der oberen Halterung befestigt, wobei der Abstand der Klemmbacken 7 cm betrug. Die Fadenausreißkraft wurde mit einer Zuggeschwindigkeit von 300 mm/min bestimmt. Die in Tabelle 3.2 wiedergegebenen Ergebnisse zeigen, dass bei dem Produkt Lyoplant® und den nach Beispiel 1 b) hergestellten Implantaten im wesentlichen gleiche Fadenausreißkräfte gemessen wurden.

Tabelle 3.2: Vergleich der mittleren Fadenausreißkraft

|  | Fadenausreißkraft | Standardabweichung |
|---|---|---|
| Implantat nach Bsp. 1 | 26,4 N | 13,4 |
| Lyoplant® | 27,3 N | 12,5 |

Im Gegensatz zu herkömmlichen selbstklebenden Collagenschwämmen besitzt das erfindungsgemäße Implantat den Vorteil, dass es vernähbar ist. Ein Vernähen ist vor allem bei größeren Defekten der Dura Mater, wie sie beispielsweise bei tumorösem Dura Mater-Gewebe auftreten, von Vorteil, da in diesen Fällen die körpereigene Dura Mater nicht vollständig als Auflagefläche für das Implantat zur Verfügung steht und das Implantat daher mit Hilfe von Nahtmaterial am Schädelknochen befestigt werden muss.

3.3 Reißkraftmessungen

Aus Lyoplant® (gefriergetrocknetes Rinderperikard B/Braun Aesculap AG, Tuttlingen) und den nach Beispiel 1 hergestellten kompositären Implantaten wurden rechteckige Streifen mit einer Länge von 100 mm und einer Breite von 25 mm zugeschnitten. Die Streifen wurden in die Klemmbacken (Abstand 7 mm) einer Zugprüfmaschine eingespannt und mit einer Zuggeschwindigkeit von 50 mm/min auseinandergezogen. Die Reißkraft wurde jeweils an n = 7 Proben bestimmt.

Tabelle 3.3: Vergleich der mittleren Reißkraft

|  | Reißkraft | Standardabweichung |
|---|---|---|
| Implantat nach Bsp. 1 | 211 N | 75 N |
| Lyoplant® | 187 N | 26 N |

Die in Tabelle 3.3 dargestellten Ergebnisse zeigen, dass die Reißkraft des erfindungsgemäßen Implantats vergleichbar mit der Reißkraft von Lyoplant® ist.
Insgesamt zeigen die in den Beispielen 3.1 bis 3.3 beschriebenen Versuche, dass die materialbedingten Eigenschaften des erfindungsgemäßen Implantats hinsichtlich seiner Stempeldurchpreßkraft, Fadenausreißkraft und Reißkraft vergleichbar mit reinem Rinderperikard sind und daher durch den schichtförmigen Aufbau des erfindungsgemäßen Implantats nicht nachteilig verändert sind. Diese Eigenschaften machen das Implantat besonders wertvoll, um beispielsweise als Ersatzmaterial für die Dura Mater, insbesondere als Dura Mater-Onlay, verwendet zu werden.

3.4 Wasseraufnahmekapazität

Aus Lyoplant® (gefriergetrocknetes Rinderperikard B/Braun Aesculap AG, Tuttlingen) und den nach Beispiel 1 hergestellten kompositären Implantaten wurden rechteckige Streifen mit einer Länge von 100 mm und einer Breite von 25 mm zugeschnitten. Die Streifen wurden anschließend in eine Petrischale mit 20 ml Wasser eingelegt. Nachdem die Implantate mit Wasser vollständig durchtränkt waren, wurde die Wasseraufnahmkapazität nach folgender Formel bestimmt:

$$X = \frac{W_{nass} - W_{trocken}}{W_{nass}} \bullet 100\%$$

Tabelle 3.4: Vergleich der mittleren Wasseraufnahmekapazität

|  | Wasseraufnahmekapazität | Standardabweichung |
|---|---|---|
| Implantat nach Bsp. 1 | 1565% | 93 % |
| Lyoplant® | 542% | 106 % |

Es stellte sich heraus, dass das erfindungsgemäße Implantat in Wasser stabil ist. Insbesondere konnte kein Ablösen der Schichten voneinander festgestellt werden. Darüber hinaus quoll weder das Produkt Lyoplant® noch das erfindungsgemäße Implantat in Wasser auf, was insbesondere für den Einsatz in der Neurochirurgie von Vorteil ist, da quellende Implantate allgemein zu Komplikationen (Schmerzen, höherer Hirndruck) führen können. Insgesamt wurden n = 6 Messungen durchgeführt.

Beispiel 4

Tierexperimentelle Untersuchungen

In einer Tierstudie mit 6 Schweinen (Tiere 1, 2, 3, 4, 5 und 6) wurde das erfindungsgemäße Implantat im Vergleich zu einem reinen Rinderperikard (ohne zusätzlichen Collagenschwamm) überprüft. Nach Narkotisierung der Tiere wurde ein ca. 6 cm langer Schnitt oberhalb des Ohrs gesetzt und die dadurch hervorgerufene Blutung durch gängige operative Methoden gestillt. Anschließend wurde mit einem chirurgischen Bohrer eine Kranioektomie mit 4 cm Durchmesser durchgeführt, um die Dura Mater der Tiere frei zu präparieren. Danach wurde in der Dura Mater ein Defekt mit einem Durchmesser von 2,5 cm gesetzt. Anschließend wurde lyophilisiertes Rinderperikard (Lyoplant® B/Braun Aesculap AG, Tuttlingen) auf die Defektgrößen der Tiere 1, 3 und 5 zugeschnitten und mit einer fortlaufenden Naht an der verbleibenden Dura Mater dieser Tiere befestigt. Die durchschnittliche Zeit von der Applikation des Rinderperikards zur intraoperativen Überprüfung der CSF-Dichtigkeit (CSF, ceresbrospinal fluid) betrug 18 Minuten. Parallel dazu wurde ein gemäß Beispiel 1 hergestelltes Implantat auf die Defektgrößen der Tiere 2, 4 und 6 zugeschnitten und anschließend auf die Defekte dieser Tiere aufgelegt. Die durchschnittliche Zeit der Applikation des Implantats bis zur intraoperativen Überprüfung der CSF-Dichtigkeit betrug 5 Minuten, was deutlich den Vorteil der Zeitersparnis für den Chirurgen belegt.
Nach erfolgter Applikation wurde mit Hilfe des sogenannten Valsalva-Manövers die Dichtigkeit des Implantats überprüft, bevor die Wunde verschlossen wurde (vgl. hierzu die Tabelle 4). Die Sektion der Tiere erfolgte nach 28 Tagen. Die Tiere wurden erneut narkotisiert und der Wundbereich der Dura Mater frei präpariert. Die Implantate (Lyoplant® sowie die erfindungsgemäßen Implantate) waren bei allen Tieren noch zu sehen. Vor der Entnahme von histologischen Proben wurde nochmals die Dichtigkeit des Dura Mater-Verschlusses mittels des Valsalva-Manövers überprüft (Tabelle 4). Sowohl der makroskopische als auch der histologische Befund zeigten dabei, dass in keinem der Tiere Adhäsionen zwischen den Hirnhäuten und dem Gehirn aufgetreten waren. Weiterhin wurde die Wundheilung und das Einwachsen der Implantate in die Dura Mater als sehr gut bewertet. Die aufgetretenen minimalen Entzündungsreaktionen waren auf die natürliche Wundheilung zurückzuführen.

Tabelle 4: Induzierter interkranialer Druck.

| Implantat | Tier Nr. | Intraoperativ CSF-dicht bis | Sektion CSF-dicht bis |
|---|---|---|---|
| Implantat nach Bsp. 1 | 2 | 2,2 kPa | 2,2 kPa |
| Implantat nach Bsp. 1 | 4 | 2,2 kPa | 2,2 kPa |
| Implantat nach Bsp. 1 | 6 | 2,2 kPa | 2,2 kPa |
| Lyoplant® | 1 | 2,2 kPa | 2,2 kPa |
| Lyoplant® | 3 | 1,1 kPa | 2,2 kPa |
| Lyoplant® | 5 | 2,2 kPa | 2,2 kPa |

Die Tierstudie zeigt, dass die Funktionalität und Sicherheit der Perikardschicht im erfindungsgemäßen Implantat vergleichbar ist mit der Funktionalität und Sicherheit des von der Anmelderin unter der Bezeichnung Lyoplant® vertriebenen Produktes. Ein besonderer Vorteil gegenüber dem kommerziell vertriebenen Produkt Lyoplant® besteht darin, dass die abdichtende Wirkung des erfindunsgemäßen Implantats in deutlich kürzerer Zeit einsetzt, woraus sich eine Zeitersparnis für den Chirurgen ergibt. Dadurch ist es letztendlich möglich, die Kosten von chirurgischen Eingriffen, insbesondere neurochirurgischen Eingriffen, zu reduzieren. Dies gilt auch im Hinblick auf größere Gewebedefekte, welche mit Hilfe des erfindungsgemäßen Implantats behandelt bzw. versorgt werden.
Insgesamt belegen die im Beispielteil der vorliegenden Anmeldung beschriebenen Versuche, dass das erfindungsgemäße Implantat aufgrund seiner besonderen materialbedingten Eigenschaften sowohl mit einem Gewebe vernäht als auch mit einem Gewebe verklebt werden kann. Das erfindungsgemäße Implantat zeichnet sich somit in besonders vorteilhafter Weise dadurch aus, dass es zum einen selbstklebend ist, zum anderen aber auch an- bzw. vernähbar ist, sofern dies aufgrund der Besonderheit des zu versorgenden Gewebedefektes gewünscht sein sollte.

**EP 2 147 687 B1**

**Patentansprüche**

1. Flächiges Implantat, insbesondere zum Ersatz von biologischen Geweben, mit einem schichtförmigen Aufbau aus

   a) einer lyophilisierten Perikardschicht biologischen Ursprungs und
   b) mindestens einer schwammartigen Schicht aus lyophilisiertem extrazellulären Protein, **dadurch gekennzeichnet, dass** das extrazelluläre Protein unvernetzt ist.

2. Flächiges Implantat nach Anspruch 1, **dadurch gekennzeichnet, dass** das extrazelluläre Protein Collagen ist.

3. Flächiges Implantat nach Anspruch 1 oder 2, **dadurch gekennzeichnet, dass** das extrazelluläre Protein Gelatine ist.

4. Flächiges Implantat nach einem der vorhergehenden Ansprüche, **dadurch gekennzeichnet, dass** die Perikardschicht einseitig mit der schwammartigen Schicht belegt ist.

5. Flächiges Implantat nach einem der vorhergehenden Ansprüche, **dadurch gekennzeichnet, dass** die der schwammartigen Schicht zugewandte Oberfläche der Perikardschicht aufgerauht ist.

6. Flächiges Implantat nach einem der vorhergehenden Ansprüche, **dadurch gekennzeichnet, dass** die schwammartige Schicht auf die Perikardschicht als Unterlage auflyophilisiert ist.

7. Implantat nach einem der vorherigen Ansprüche, **dadurch gekennzeichnet, dass** die Perikardschicht eine Dicke zwischen 0,1 und 4 mm, vorzugsweise 0,5 und 2 mm, aufweist.

8. Flächiges Implantat nach einem der vorhergehenden Ansprüche, **dadurch gekennzeichnet, dass** die schwammartige Schicht eine Schichtdicke von 1 bis 10, inbesondere 2 bis 5, vorzugsweise 3 bis 4 mm, besitzt.

9. Flächiges Implantat nach einem der vorhergehenden Ansprüche, **dadurch gekennzeichnet, dass** die Perikardschicht ein Flächengewicht von 50 bis 180 g/m$^2$ besitzt.

10. Flächiges Implantat nach einem der vorhergehenden Ansprüche, **dadurch gekennzeichnet, dass** die schwammartige Schicht ein Flächengewicht von 30 bis 300 g/m$^2$ besitzt.

11. Flächiges Implantat nach einem der vorherigen Ansprüche, **dadurch gekennzeichnet, dass** die schwammartige Schicht ein Flüssigkeitsaufnahmevermögen aufweist, das dem 20- bis 60-flachen, insbesondere 30- bis 60-fachen, seines Eigengewichtes entspricht.

12. Flächiges Implantat nach einem der vorherigen Ansprüche, **dadurch gekennzeichnet, dass** die schwammartige Schicht innerhalb eines Zeitraums < 100 s, insbesondere < 60 s, vollständig mit Wasser benetzbar ist.

13. Flächiges Implantat nach einem der vorhergehenden Ansprüche als Implantat zum Ersatz und/oder Verschluss der Dura Mater, vorzugsweise als Dura Mater-Onlay.

14. Verfahren zur Herstellung eines Implantats, wobei eine Perikardschicht und zumindest eine Schicht aus extrazellulärem Protein unter Verbindung miteinander lyophilisiert werden, **dadurch gekennzeichnet, dass** das extrazelluläre Protein unvernetzt ist.

15. Verwendung von a) einer lyophilisierten Perikardschicht biologischen Ursprungs und b) mindestens einer schwammartigen Schicht aus lyophilisiertem extrazellulären Protein zur Herstellung eines flächigen Implantats, **dadurch gekennzeichnet, dass** das extrazelluläre Protein unvernetzt ist.

**Claims**

1. Two-dimensional implant, in particular for replacement of biological tissues, with a layered structure composed of

   a) a lyophilized pericardium layer of biological origin, and
   b) at least one sponge-like layer of lyophilized extracellular protein, **characterized in that** the extracellular protein is uncrosslinked.

2. Two-dimensional implant according to Claim 1, **characterized in that** the extracellular protein is collagen.

3. Two-dimensional implant according to Claim 1 or 2, **characterized in that** the extracellular protein is gelatin.

4. Two-dimensional implant according to any of the preceding claims, **characterized in that** the pericardium layer is covered on one side with the sponge-like layer.

5. Two-dimensional implant according to any of the preceding claims, **characterized in that** the surface of the pericardium layer directed toward the sponge-like layer is roughened.

6. Two-dimensional implant according to any of the preceding claims, **characterized in that** the sponge-like layer is lyophilized as substrate onto the pericardium layer.

7. Implant according to any of the preceding claims, **characterized in that** the pericardium layer has a thickness of between 0.1 and 4 mm, preferably of between 0.5 and 2 mm.

8. Two-dimensional implant according to any of the preceding claims, **characterized in that** the sponge-like layer has a layer thickness of 1 to 10 mm, in particular of 2 to 5 mm, preferably of 3 to 4 mm.

9. Two-dimensional implant according to any of the preceding claims, **characterized in that** the pericardium layer has a weight per unit area of 50 to 180 $g/m^2$.

10. Two-dimensional implant according to any of the preceding claims, **characterized in that** the sponge-like layer has a weight per unit area of 30 to 300 $g/m^2$.

11. Two-dimensional implant according to any of the preceding claims, **characterized in that** the sponge-like layer has a liquid absorption capacity corresponding to 20 to 60 times, in particular 30 to 60 times its own weight.

12. Two-dimensional implant according to any of the preceding claims, **characterized in that** the sponge-like layer can be completely wetted with water within a period of < 100 seconds, in particular of < 60 seconds.

13. Two-dimensional implant according to any of the preceding claims as an implant for replacement and/or closure of the dura mater, preferably as a dura mater onlay.

14. Method for producing an implant, wherein a pericardium layer and at least one layer of extracellular protein are lyophilized and connected to each other, **characterized in that** the extracellular protein is uncrosslinked.

15. Use of a) a lyophilized pericardium layer of biological origin and b) at least one sponge-like layer of lyophilized extracellular protein for producing a two-dimensional implant, **characterized in that** the extracellular protein is uncrosslinked.

**Revendications**

1. Implant bidimensionnel, en particulier pour replacement des tissus biologiques, ayant une structure stratifiée, constitué

   a) d'une couche péricardique lyophilisée d'origine biologique, et
   b) d'au moins une couche spongieuse, constituée d'une protéine extra-cellulaire lyophilisée, **caractérisé en ce que** la protéine extra-cellulaire n'est pas réticulée.

2. Implant bidimensionnel selon la revendication 1, **caractérisé en ce que** la protéine extra-cellulaire est le collagène.

3. Implant bidimensionnel selon la revendication 1 ou 2, **caractérisé en ce que** la protéine extra-cellulaire est la gélatine.

**4.** Implant bidimensionnel selon l'une des revendications précédentes, **caractérisé en ce que** la couche péricardique est, sur une face, occupée par la couche spongieuse.

**5.** Implant bidimensionnel selon l'une des revendications précédentes, **caractérisé en ce que** la surface de la couche péricardique en regard de la couche spongieuse est grattée.

**6.** Implant bidimensionnel selon l'une des revendications précédentes, **caractérisé en ce que** la couche spongieuse est appliquée par lyophilisation sur la couche péricardique, sous forme d'un substrat.

**7.** Implant selon l'une des revendications précédentes, **caractérisé en ce que** la couche péricardique présente une épaisseur comprise entre 0,1 et 4 mm, de préférence entre 0,5 et 2 mm.

**8.** Implant bidimensionnel selon l'une des revendications précédentes, **caractérisé en ce que** la couche spongieuse présente une épaisseur de couche de 1 à 10 mm, en particulier de 2 à 5 mm, de préférence de 3 à 4 mm.

**9.** Implant bidimensionnel selon l'une des revendications précédentes, **caractérisé en ce que** la couche péricardique présente une aire surfacique de 50 à 180 $g/m^2$.

**10.** Implant bidimensionnel selon l'une des revendications précédentes, **caractérisé en ce que** la couche spongieuse présente une masse surfacique de 30 à 300 $g/m^2$.

**11.** Implant bidimensionnel selon l'une des revendications précédentes, **caractérisé en ce que** la couche spongieuse présente une capacité d'absorption des liquides qui correspond à 20 à 60 fois, en particulier à 30 à 60 fois son poids propre.

**12.** Implant bidimensionnel selon l'une des revendications précédentes, **caractérisé en ce que** la couche spongieuse peut être complètement mouillée à l'eau sur un laps de temps < 100 secondes, en particulier < 60 secondes.

**13.** Implant bidimensionnel selon l'une des revendications précédentes, servant d'implant pour remplacement et/ou fermeture de la dure-mère, de préférence sous forme d'une greffe apposée de la dure-mère.

**14.** Procède de fabrication d'un implant, dans lequel une couche péricardique et au moins une couche constituée de protéine extra-cellulaire sont lyophilisées en étant assemblées l'une à l'autre, **caractérisé en ce que** la protéine extra-cellulaire n'est pas réticulée.

**15.** Utilisation a) d'une couche péricardique lyophilisée d'origine biologique, et b) d'au moins une couche spongieuse constituée d'une protéine extra-cellulaire lyophilisée, pour la fabrication d'un implant bidimensionnel, **caractérisée en ce que** la protéine extra-cellulaire n'est pas réticulée.

Fig. 1

10

12

13

Fig. 2

20

22

23

Fig. 3

30

33

32